# EUROPÄISCHE PATENTSCHRIFT

(11) **EP 2 040 692 B1**
(45) Veröffentlichungstag und Bekanntmachung des Hinweises auf die Patenterteilung: **19.09.2012**
(21) Anmeldenummer: 07787591.2
(22) Anmeldetag: 16.07.2007
(51) Int. Cl.: A61K 31/198, A61K 31/405, A61K 36/23, A61K 36/28, A61K 36/888, A61K 36/185, A61K 36/38, A61K 36/48, A61K 36/53, A61K 36/482, A61K 36/35, A61K 31/04, A61P 31/10, A61P 31/12

(54) **MEDIZINISCHE VERWENDUNG VON N-PHENYLPROPENOYL-AMINOSÄUREDERIVATEN**
MEDICAL USE OF N-PHENYLPROPENOYL-AMINO ACID DERIVATIVES
UTILISATION MÉDICALE DES DÉRIVÉS D'ACIDES N-PHÉNYLPROPÉNOYL-AMINÉS

(30) Priorität: 17.07.2006 DE 102006033321
(43) Veröffentlichungstag der Anmeldung: 01.04.2009
(73) Patentinhaber: Westfälische Wilhelms-Universität Münster, 48149 Münster (DE)
(72) Erfinder: HOFMANN, Thomas, 85375 Neufahrn (DE); DETERS, Alexandra, 48161 Münster (DE); STARK, Timo, 85402 Kranzberg (DE); HENSEL, Andreas, 48161 Münster (DE)
(74) Vertreter: Michalski Hüttermann & Partner
(86) Internationale Anmeldenummer: PCT/EP2007/057324
(87) Internationale Veröffentlichungsnummer: WO 2008/009655

(56) Entgegenhaltungen:
- EP-A- 0 226 304
- EP-A- 0 815 833
- WO-A-00/61545
- WO-A-99/42435
- WO-A-99/48371
- WO-A-03/071884
- WO-A-03/089405
- WO-A-2004/062601
- WO-A-2005/072723
- US-A- 4 666 890
- US-A- 5 700 821
- US-A1- 2003 186 967
- US-A1- 2006 067 990
- REINKE RYAN A ET AL: "Dicaffeoyltartaric acid analogues inhibit human immunodeficiency virus type 1 (HIV-1) integrase and HIV-1 replication at nontoxic concentrations." JOURNAL OF MEDICINAL CHEMISTRY 15 AUG 2002, Bd. 45, Nr. 17, 15. August 2002 (2002-08-15), Seiten 3669-3683, XP007904031 ISSN: 0022-2623
- CHARVAT ET AL: "Design, synthesis, and biological evaluation of chicoric acid analogs as inhibitors of HIV-1 integrase" BIOORGANIC & MEDICINAL CHEMISTRY, ELSEVIER SCIENCE LTD, GB, Bd. 14, Nr. 13, 1. Juli 2006 (2006-07-01), Seiten 4552-4567, XP005445095 ISSN: 0968-0896
- NOMURA EISAKU ET AL: "Synthesis of amide compounds of ferulic acid, and their stimulatory effects on insulin secretion in vitro" BIOORGANIC & MEDICINAL CHEMISTRY, ELSEVIER SCIENCE LTD, GB, Bd. 11, Nr. 17, 15. August 2003 (2003-08-15), Seiten 3807-3813, XP002425367 ISSN: 0968-0896
- MIAO Z ET AL: "Synthesis and biological activities of ferulic acid-amino acid derivatives" BIOSCIENCE BIOTECHNOLOGY BIOCHEMISTRY, JAPAN SOC. FOR BIOSCIENCE, BIOTECHNOLOGY AND AGROCHEM, TOKYO, JP, Bd. 61, Nr. 3, 1997, Seiten 527-529, XP009095338 ISSN: 0916-8451
- TAKEUCHI H ET AL: "FURTHER STUDY ON EFFECTS OF N-BETA PHENYLPROPIONYL-L TYROSINE AND ITS DERIVATIVES ON THE EXCITABILITY OF AN IDENTIFIABLE GIANT NEURON OF ACHATINA-FULICA" COMPARATIVE BIOCHEMISTRY AND PHYSIOLOGY C PHARMACOLOGY TOXICOLOGY AND ENDOCRINOLOGY, Bd. 75, Nr. 2, 1983, Seiten 329-336, XP002468364 ISSN: 0742-8413
- OSAKABE, N.; YAMAGISHI, M.; NATSUME, M.; TAKIZAWA, T.; NAKAMURA, T.; OSAWA, T.: "Antioxidative polyphenolic substances in cacao liquor" ACS SYMPOSIUM SERIES, 2000, XP009095708
- STARK TIMO ET AL: "Isolation, structure determination, synthesis, and sensory activity of N-phenylpropenoyl-L-amino acids from cocoa (Theobroma cacao)." JOURNAL OF AGRICULTURAL AND FOOD CHEMISTRY 29 JUN 2005, Bd. 53, Nr. 13, 29. Juni 2005 (2005-06-29), Seiten 5419-5428, XP007903956 ISSN: 0021-8561 in der Anmeldung erwähnt
- PUROHIT ET AL: "Preparation and antimicrobial evaluation of 1,1,1-trichloro-2,2- bis(carboxymethylaminocarbonylaryl)ethanes as potent new DDT analogs" HETEROCYCLIC COMMUNICATIONS, FREUND PUBLISHING HOUSE, TEL AVIV, IL, Bd. 3, Nr. 5, 1997, Seiten 437-441, XP009095359 ISSN: 0793-0283
- OTANI, THEODORE T. ET AL: "N-Benzoyl derivatives of amino acids and amino acid analogs as growth inhibitors in microbial antitumor screen" JOURNAL OF PHARMACEUTICAL SCIENCES , 68(11), 1366-9 CODEN: JPMSAE; ISSN: 0022-3549, 1979, XP002469800
- DATABASE CA [Online] CHEMICAL ABSTRACTS SERVICE, COLUMBUS, OHIO, US; VACIRCA, F. ET AL: "Antimicrobic activity of some derivatives of aminovaleric and aminocaproic acids" XP002469801 gefunden im STN Database accession no. 1946:24139 & BOLLETTINO DELL'ISTITUTO SIEROTERAPICO MILANESE , 23, 99-102 CODEN: BISMAP; ISSN: 0021-2547, 1944,
- DATABASE WPI Week 200542 Derwent Publications Ltd., London, GB; AN 2005-417831 XP002469805 & WO 2005/051340 A (SHISEIDO CO LTD) 9. Juni 2005 (2005-06-09)
- DATABASE CA [Online] CHEMICAL ABSTRACTS SERVICE, COLUMBUS, OHIO, US; MAY, RAOUL M.: "Effect of dibenzoylcystine on the cicatrization of mouse skin: new technique for its study" XP002469802 gefunden im STN Database accession no. 1959:106799 & COMPT. REND. , 247, 1670-3, 1958,
- SCOZZAFAVA A ET AL: "ANTIMYCOBACTERIAL ACTIVITY OF 3,4-DICHLOROPHENYL-UREAS, N,N-DIPHENYL-UREAS AND RELATED DERIVATIVES" JOURNAL OF ENZYME INHIBITION, NEW YORK, NY, US, Bd. 16, Nr. 5, 2001, Seiten 425-432, XP008051962
- DATABASE CA [Online] CHEMICAL ABSTRACTS SERVICE, COLUMBUS, OHIO, US; SPIES, TOM D. ET AL: "Ten year study of synthetic folic acid as a nutritional supplement and therapeutic agent" XP002468365 gefunden im STN Database accession no. 1957:82293 & GP, J. AM. ACAD. GEN. PRACT. , 16(NO. 2), 85-103, 1957,
- REZNIK, L. V. ET AL: "Succinate dehydrogenase activity in rat kidney after repeated administrations of p-aminohippurate or sodium chloride" ACTA BIOLOGICA ET MEDICA GERMANICA , 38(8), 1135-9 CODEN: ABMGAJ; ISSN: 0001-5318, 1979, XP009096311
- KHAN T H ET AL: "Novel inhibitors of carboxypeptidase G2 (CPG2): potential use in antibody-directed enzyme prodrug therapy." JOURNAL OF MEDICINAL CHEMISTRY 25 MAR 1999, Bd. 42, Nr. 6, 25. März 1999 (1999-03-25), Seiten 951-956, XP002469803 ISSN: 0022-2623
- DATABASE CA [Online] CHEMICAL ABSTRACTS SERVICE, COLUMBUS, OHIO, US; NAKAMIZO, NOBUHIRO ET AL: "Thiocarbonylamino acids as microbiocides" XP002469804 gefunden im STN Database accession no. 1979:181578 & JP 53 148530 A (KYOWA HAKKO KOGYO CO., LTD., JAPAN) 25. Dezember 1978 (1978-12-25)

## Beschreibung

Die vorliegende Erfindung betrifft die medizinische, kosmetische und lebensmitteltechnische Verwendung von N-Phenylpropenoyl-Aminosäurederivaten und verwandten Verbindungen. Bei diesen Verbindungen handelt es sich um sekundäre Pflanzenstoffe, die insbesondere aus der Kakaopflanze (*Theobroma cacao*) isoliert werden können.

Der gesundheitliche Nutzen von sekundären Pflanzenstoffen ist seit langem bekannt. Für eine Vielzahl von Stoffen wurden antimikrobielle oder antioxidative Eigenschaften beschrieben, so z.B. für Polyphenole wie das Epikatechin, das aus der Kakaopflanze gewonnen wird.

Bekannt ist ebenso die Rosmarinsäure. Hierbei handelt es sich um einen 3-(3,4-Dihydroxy-phenyl)-acrylsäure-1-carboxy-2-(3,4-dihydroxy-phenyl)-ethylester. Diese Verbindung sowie deren Derivate weisen bemerkenswerte Eigenschaften auf, so z.B. antibakterielle, antivirale, antiinflammatorische, antigonadotrope und antioxidative Eigenschaften.

Unter physiologischen Bedingungen werden diese Verbindungen jedoch durch körpereigene Esterasen relativ schnell hydrolysiert, so dass bei einer Verabreichung nur geringe Mengen in den Blutkreislauf gelangen und die Verweildauer nur sehr kurz ist. Es können daher nur niedrige Bluttiter erreicht werden, die oftmals unter der für einen möglichen therapeutischen Nutzen erforderlichen Schwelle liegen.

Eine neue Gruppe sekundärer Pflanzenstoffe, die N-Phenylpropenoyl-Aminosäurederivate, wurden erstmals in dem Artikel [1] von Stark und Hofmann (2005), "Isolation, structure determination, synthesis, and sensory activity of N-phenylpropenoyl-L-amino acids from cocoa (Theobroma cocoa)", J. Agric. Food Chem; 53: 5419-5428, beschrieben. Diese Verbindungen bestehen aus einem ggf. substituierten Phenyl-Propensäure-Rest, der über eine Amidbindung mit einem Aminosäurerest verbunden ist.

Aufgabe der vorliegenden Erfindung ist es, sekundäre Pflanzenstoffe mit medizinischen Indikationen bereitzustellen, die eine höhere Verweildauer im Organismus aufweisen und ähnliche oder bessere oder zusätzliche medizinische, kosmetische und lebensmitteltechnische Eigenschaften aufweisen wie bzw. als bereits bekannte sekundäre Pflanzenstoffe.

Diese Aufgabe wird mit den Merkmalen des vorgelegten Hauptanspruchs gelöst. Die Unteransprüche zeigen bevorzugten Ausführungsformen.

Demnach ist die Verwendung von Verbindungen der allgemeinen Strukturformel 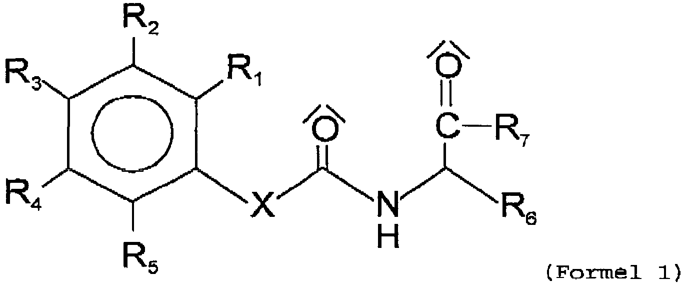 wobei es sich bei diesen Verbindungen um E-konfigurierte N Phenylpropenoyl-L-Aminosäurederivate handelt, und
wobei es sich bei x um einen HC=CH-Rest handelt,
wobei es sich bei R5, R4 und R1 um Wasserstoffreste (H-)handelt,
wobei es sich bei R3 um eine Hydroxigruppe (HO) handelt,
wobei es sich bei R2 um einen Wasserstoffrest (H-) oder eine Hydroxigruppe (HO) handelt,
wobei es sich bei R6 um einen -CH2-COOH-Rest oder einen - CH2-CH2-COOH-Rest handelt, und
wobei es sich bei R7 um eine Hydroxigruppe (-OH) handelt,
in einem chirurgischen, therapeutischen oder diagnostischen Verfahren zur Behandlung des menschlichen oder tierischen Körpers vorgesehen.

Eine solche medizinische Verwendung von Verbindungen, die unter diese allgemeine Formel fallen, wird in der vorliegenden Erfindung erstmals beschrieben. Bezüglich experimenteller Hinweise auf mögliche medizinische Indikationen wird auf die Beispiele verwiesen.

Diese Verbindungen weisen strukturell große Ähnlichkeiten mit häufig in der natur vorkommenden substituierten Zimtsäureestern (Rosmarinsäure, Chlorogensäure, etc.) bzw. ihren Derivaten auf. Allerdings sind die beiden Grundmoleküle bei den erfindungsgemäßen Verbindungen über eine Amidbindung verknüpft, die unter physiologischen Bedingungen sehr viel stabiler ist als die Esterbindung bei den substituierten Zimtsäureestern., die wie bereits erwähnt durch die ubiquitären Esterasen im Körper schnell hydrolysiert werden.

Voraussetzung für die physiologischen Effekte und damit die Eignung für etwaige medizinische Indikationen ist mit großer Wahrscheinlichkeit einerseits die Amidbindung, die insbesondere unter physiologischen Bedingungen sehr viel stabiler ist als z.B. eine Esterbindung, und daher insbesondere die Aufnahme der erfindungsgemäßen Verbindungen durch das Dünndarmepithel in den Blutkreislauf bzw. eine ausreichend lange Verweildauer der erfindungsgemäßen Verbindungen im Blutkreislauf ermöglicht.

Andererseits scheint das Vorhandensein der Carboxylgruppe, die durch die Aminosäure, die Hydroxigruppe an R₇ bereitgestellt wird, eine Voraussetzung für die physiologische Wirksamkeit zu sein.

Es handelt sich bei der erfindungsgemäßen Verbindung um N-Phenylpropenoyl-Aminosäure derivate. Eine solche Verbindung hat die folgende allgemeine Strukturformel:

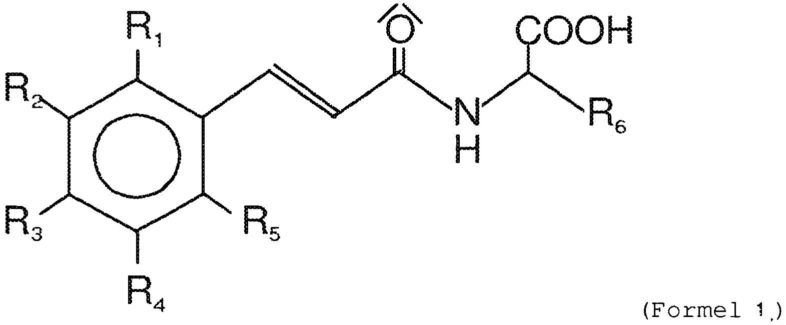

Chemisch gesehen handelt es sich hierbei um eine Amidverbindung aus einer ggf. substituierten Zimtsäure (Phenyl-Propensäure bzw. Phenyl-Acrylsäure) und einer Aminosäure. Ist der Phenoylrest an R₂ und R₃ mit je einer Hydroxigruppe substituiert, handelt es sich um Kaffeesäure. Die Amidbindung ist ausgebildet zwischen der Aminogruppe der Aminosäure und der Carboxylgruppe der Zimtsäure bzw. Kaffeesäure.

Weiterhin ist die Verwendung einer Verbindung gemäß Formel (1) zur Herstellung eines Arzneimittels zur Vorbeugung und/oder Behandlung von bakteriellen, viralen oder mykologischen Infektionen vorgesehen.

Untersuchungen der Erfinder haben überraschenderweise gezeigt, dass die erfindungsgemäßen Verbindungen antiadhäsive Wirkung gegenüber der Ansiedlung von Bakterien, Viren und Pilzen auf Oberflächen wie z.B. der Haut, den Schleimhäuten, der Speiseröhre, der Magenwand und dem Dünndarmepithel aufweisen.

Diese Wirkung wurde beispielhaft an *Helicobacter pylori* auf der Magenschleimhaut gezeigt. Hierzu wird auf die Beispiele verwiesen. Es liegen zudem Hinweise für eine antiadhäsive Wirkung gegen *Campylobacter jejuni,* adhärente *E. coli, Porphyromonas gingivalis, Staphylococcus aureus* und *Candida albicans* vor. Diese Ergebnisse lassen auf eine allgemeine antiadhäsive Wirkung gegen Mikrororganismen schließen, insbesondere gegen gram-positive und gramnegative Bakterien, Viren und Pilzen.

Eine Ursache für diese Wirkung scheint darin zu liegen, dass die erfindungsgemäßen Verbindungen die Ausbildung einer mikrobiellen Ansiedlungsmatrix (häufig bestehend aus Polysacchariden und Glycoproteinen) hemmen oder dass sie in Wechselwirkungen mit mikrobiellen, insbesondere bakteriellen Adesinen treten oder die für die Adhäsion zuständigen Rezeptorfunktionen auf der Epithelseite der zu besiedelnden Oberfläche blockieren.

Diese Eigenschaften könnten z.B. für die Herstellung eines Medikaments zur Behandlung und Prophylaxe chronischer Magenschleimhautentzündung oder für die Infektionsprävention, z.B. bei Hautverbrenungen,oder bei schlecht heilenden Wunden (z.B. Ulcus) von Nutzen sein.

Weitere potentielle Indikationen erschließen sich dem Fachmann unmittelbar.

Die Verwendung einer Verbindung gemäß Formel (1) zur Herstellung eines Arzneimittels zur hepalen Regeneration, zur Verbesserung des Zellstoffwechsels und/oder zur Verbesserung der Zellproliferation ist somit ebenfalls eine bevorzugte Ausführungsform der Erfindung.

In diesem Zusammenhang haben Experimente der Erfinder gezeigt, dass die erfindungsgemäßen Verbindungen eine proliferationsfördernde Wirkung auf humane Keratinocyten ausüben, ohne dass dabei die Expression zellulärer Wachstumsfaktoren beeinflusst wird. Hierzu wird auf die Beispiele verwiesen.

Die Erfinder haben gezeigt, dass die Verbindungen deutliche Wirkungen auf humane Leberzellen haben und die Energieproduktion sowie die Zellproliferation deutlich zu steigern in der Lage sind. Weiterhin wurde gezeigt, dass die erfindungsgemäßen Verbindungen eine Erhöhung der mitochondrialen Aktivität von Leberzellen hervorrufen. Hierzu wird auf die Beispiele verwiesen.

Mögliche Indikationen sind in diesem Zusammenhang die Regeneration von Leberzellen, die durch Chemotherapeutika, Strahlenbehandlung, Arzneimittelbehandlung, Arneimittelintoxikation, Alkoholmißbrauch, Drogenmißbrauch, Vergiftungen (insb. Pilzvergiftungen), Leberinfektionen (insb. Hepatitis) geschädigt wurden.

Weitere Anwendungsmöglichkeiten sind eine Erhöhung der mitochondrialen Aktivität, insbesondere von Cytochrom P450, das als ein zelluläres Entgiftungsenzym wirkt.

Weitere Indikationen sind der Einsatz der erfindungsgemäßen Verbindungen zur Herstellung eines Arzneimittels zur Förderung der Wundheilung, der Hautregeneration, der Schleimhautregeneration, der erhöhten Proliferation von Hautanhangsgebilden (z.B. Haare) und der Knorpelbildung, zur Prävention und Therapie von Dekubitus und Narbenbildung, oder zur Haut- und Geweberegeneration z.B. nach Verbrennungen, Verätzungen, Wundliegen etc.

Aufgrund der antiadhäsiven Wirkung ist eine Verwendung einer erfindungsgemäßen Verbindung zur Herstellung eines Arzneimittels gegen orale Plaque ebenfalls denkbar.

Weiterhin ist erfindungsgemäß vorgesehen, eine Verbindung gemäß einem der vorherigen Ansprüche als Nahrungsergänzungsmittel und "functional food" zu verwenden.

Hier kommt neben den genannten Wirkungen (antiadhäsiv, zellproliferationsfördernd, zellstoffwechselerhöhend) zum Tragen, dass die erfindungsgemäßen Verbindungen wie viele sekundäre Pflanzenstoffe mit großer Wahrscheinlichkeiteine antioxidative Wirkung haben.

Hinzu kommt, dass die erfindungsgemäßen Verbindungen wasserlöslich sind und sich daher in hohen Konzentrationen auch fettarmen, fettfreien oder kalorienreduzierten Lebensmitteln beimischen lassen und dass sie einfach zu isolieren bzw. synthetisieren sind. All diese Eigenschaften lassen die erfindungsgemäßen Verbindungen für die Verwendung in sog. "functional food" und als Nahrungsergänzungsmittel geeignet erscheinen.

Weiterhin ist die Verwendung einer erfindungsgemäßen Verbindung als Beigabe zu einem Zellkulturmedium vorgesehen. Die erwähnte zellproliferationsfördernde Wirkung lässt die erfindungsgemäßen Verbindungen insbesondere für die Verwendung in In-vitro-Zellvermehrungskulturen als geeignet erscheinen, insbesondere bei der Herstellung künstlicher Gewebe und Organe, für Hautmodelle oder autologe Implantationssysteme. Die zellproliferationsfördernde Wirkung scheint dabei sowohl humane, tierische als auch pflanzliche Zellkulturen zu betreffen.

Ein Erklärungsansatz hierfür könnte sein, dass die erfindungsgemäßen Verbindungen natürlicher Weise insbesondere in Pflanzensamen (wie z.B. Kakaobohnen) vorkommen, und dort die Zellteilung nach der Keimung fördern.

Aufgrund der oben erwähnten antiadhäsiven Wirkung ist außerdem bevorzugt die Verwendung einer erfindungsgemäßen Verbindung zur Verhinderung der Ausbildung von mikrobiellen Belägen auf Oberflächen vorgesehen.

Hier ist insbesondere an Implantate, Prothesen, Katheter (insbesondere Pulmonal- und Blasenkathether), Kanülen, chirurgische und diagnostische Instrumente (insbesondere Endoskope) und Zahnprothesen gedacht. Auf diese Weise könnte insbesondere der Verschleppung von häufig gegen Antibiotika resistenten Krankenhauskeimen begegnet werden.

Weitere Verwendungsgebiete sind Abwasserrohre, Rohrleitungen für Lebensmittel wie z.B. Milch, Rohrleitungen in sanitären Anlagen und Schwimmbädern (insbesondere Whirlpools), Antifoulinganstriche für Schiffe, Oberflächen von Windeln, Pflastern und anderer Hygieneartikel sowie kosmetische Instrumente, wie z.B. Zahnbürsten,

Aufgrund der hohen chemischen Stabilität sind die erfindungsgemäßen Verbindungen gerade für diese Einsatzbereiche an exponierten Oberflächen sehr gut geeignet.

Weiterhin ist die Verwendung einer erfindungsgemäßen Verbindung als Haut- oder Mundpflegemittel bevorzugt vorgesehen. Hierbei ist an Hautcremes, Antiageing-Produkte, Produkte zur Verhinderung der Narbenbildung oder zur Behandlung von Verbrennungen und Sonnenbränden, Zahncremes, Mundspülungen und dergleichen gedacht. Je nach Verwendung steht dabei die antiadhäsive, die zellproliferationsfördernde, die zellstoffwechselerhöhende oder die antioxidative Wirkung der erfindungsgemäßen Verbindungen im Mittelpunkt.

Ferner ist erfindungsgemäß ein Arzneimittel, Kosmetikum, Hautpflegemittel, Mittel zur Behandlung von Oberflächen, oder ein Nahrungsergänzungsmittel oder Functional food vorgesehen, das eine erfindungsgemäße Verbindung enthält.

Überdies ist eine diagnostische, therapeutische oder kosmetische Darreichungsform vorgesehen, die eine erfindungsgemäße Verbindung enthält, und in Form eines wässrigen Extrakts, einer Lösung, einer Emulsion, einer Suspension, eines pulmonalen Inhalates, eines Implantates, einer Wasser/Öl-Emulsion, eine Öl/Wasser-Emulsion, eines Gels, einer Tablette, einer Kapsel, einer Creme, einer salbe, eines Pflasters, einer Mikroemulsion, einer Nanoemulsion, als Transferosome oder enkapsuliert in Liposomen, Micellen oder Microsphären vorliegt.

Zur Isolierung und Aufarbeitung einer erfindungemäßen Verbindung ist ein Verfahren vorgesehen, aufweisend die Schritte der Gewinnung von Pflanzenmaterial, Herstellung eines wässrigen oder eines wässrigen-alkoholischen Extraktes, Zentrifugation des Extrakts, ggf. Lyophilisierung des Extrakts, und Aufreinigung des Extrakts mittels chromatographischer Verfahren (z.B. IEC, GPC, Adsorptionschromatographie, Verteilungschromatographie) und/oder Ultrafiltratonsverfahren bis zu einer Konzentration von mindestens 1 g pro 100 g Extrakt vorgesehen.

Bei dem der Extraktion zu unterziehenden Pflanzenmaterial handelt es sich bevorzugt um Pflanzenmaterial von Pflanzen aus der weiter unten aufgeführten Liste. Für die wässrige Extraktion kann dabei z.B. 1 g getrocknetes Pflanzenmaterial 2 x 15 min mit 15 ml zweifach destilliertem Wasser inkubiert werden. Die Zentrifugation kann z.B. 10 min lang bei 5000 x g erfolgen.

Es sei im Weiteren erwähnt, dass die Erfinder auch ein Syntheseverfahren zur Herstellung der erfindungsgemäßen Verbindungen entwickelt haben. Hierzu wird auf die Publikation [1] verwiesen.

### Zeichnungen und Beispiele

Die vorliegende Erfindung wird durch die im Folgenden gezeigten und diskutierten Beispiele und Figuren genauer erläutert. Dabei ist zu beachten, dass die Beispiele und Figuren nur beschreibenden Charakter haben und nicht dazu gedacht sind, die Erfindung in irgendeiner Form einzuschränken.

### Tabelle 1

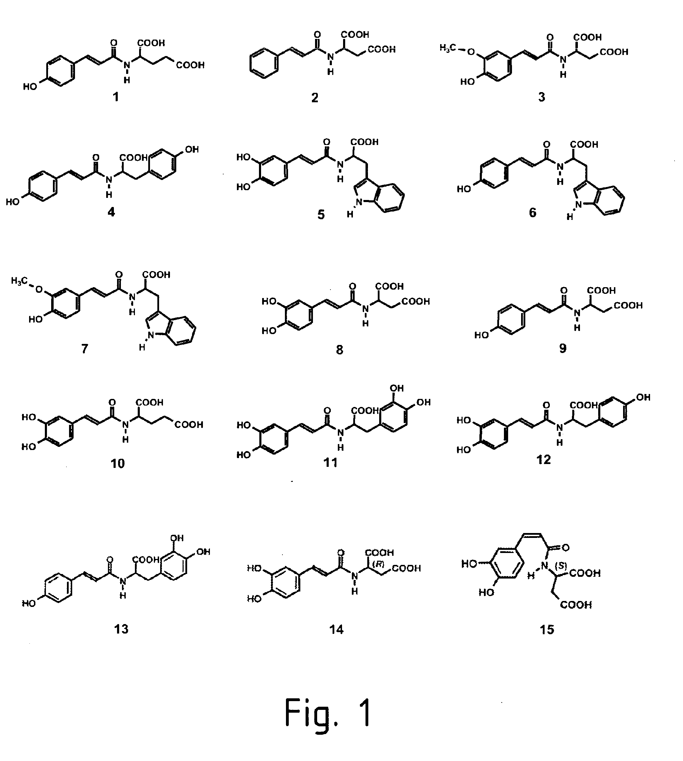

In Tabelle 1 sind verschiedene Verbindungen gezeigte , die aus verschiedenen Pflanzen isoliert wurden. Die Strukturformeln derselben Verbindungen sind in Fig. 1 gezeigt. Es handelt sich hierbei durchweg um N-Phenylpropenoyl-Aminosäuren . Die chemischen Bezeichnungen gehen aus Tabelle 1 hervor.

Die verbindungen 1 und 8-10 fallen unter den Schutzbereich der vorliegenden Erfindung.

Die verbindungen 2-7 und 11-15 sind Referenzverbindungen.

**Tabelle 1**

| **Substanz** | **Nr.** |
|---|---|
| (-)- N-[4'-hydroxy-(E)-cinnamoyl]-L-Glutaminsäure | 1 |
| (+)- N-[(E)-cinnamoyl]-L-Asparaginsäure | 2 |
| (+)- N-[4'-hydroxy-3-methoxy-(E)-cinnamoyl]-L- Asparaginsäure | 3 |
| (-)- N-[4'-hydroxy-(E)-cinnamoyl]-L-Tyrosin | 4 |
| (+)- N-[3',4'-dihydroxy-(E)-cinnamoyl]-L-Tryptophan | 5 |
| (+)- N-[4'-hydroxy-(E)-cinnamoyl]-L-Tryptophan | 6 |
| (+)- N-[4'-hydroxy-3-methoxy-(E)-cinnamoyl]-L-Tryptophan | 7 |
| (+)- N-[3',4'-dihydroxy-(E)-cinnamoyl]-L- Asparaginsäure | 8 |
| (+)- N-[4'-hydroxy-(E)-cinnamoyl]-L- Asparaginsäure | 9 |
| (-)- N-[3',4'-dihydroxy-(E)-cinnamoyl]-L-Glutaminsäure | 10 |
| (-)- N-[3',4'-dihydroxy-(E)-cinnamoyl]-3-hydroxy-L-Tyrosin | 11 |
| (-)- N-[3',4'-dihydroxy-(E)-cinnamoyl]-L-Tyrosin | 12 |
| (-)- N-[4'-hydroxy-(E)-cinnamoyl]-3-hydroxy-L-Tyrosin | 13 |
| (-)- N-[3',4'-dihydroxy-(E)-cinnamoyl]-D- Asparaginsäure | 14 |
| (+)- N-[3',4'-dihydroxy-(Z)-cinnamoyl]-L- Asparaginsäure | 15 |

### Beispiel 1

Getrocknetes Material der in Liste 1 aufgeführten Pflanzen wurde einer wässrigen Extraktion gemäß dem oben beschriebenen Verfahren unterzogen. Die gewonnenen Extrakte wurden dann einer HPLC (HochdruckFlüssigkeitschromatographie), einer LCMS (Flüssigkeitschromatographie/Massenspektrometrie) bzw. einer NMR (KernspiN-Resonanzspektrometrie) unterzogen. Hierbei wurden synthetisierte, mit Deuterium markierte Analoga der Verbindungen 2, 4 5, 7, 8, 9, 11 und 12 aus Tabelle 1 als Marker mitgemessen.

### Liste 1

| |
|---|
| *Acorus calamus, Angelica archangelica, Arnica montana, Arnica chamissonis, Betula spec., Cassia angustifolia, Cassia senna, Cinnamomum ceylanicum, Cola nitida, Coriandrum sativum, Crocus sativus, Eucalyptus spec., Gentiana lutea, Hedera helix, Hypericum perforatum, Ilex paraguariensis, Illicum verumm, Juniperus communis, Lavandula spec., Matricaria recutita, Pausinystalia yohimbe, Physostigma venenosum, Primula veris, Primula elatior, Ricinus communis, Salix sp., Sambucus nigra, Silybum marianum, Syzygium aromaticum, Theobroma cacao, Thymus vulgaris, Thymus zygis, Trigonella foenum-graecum* |

In folgenden Pflanzen konnten die Verbindungen nachgewiesen werden (Nummerierung der Verbindungen gemäß Tabelle 1):

**Tabelle 2**

| **Pflanze** | **Teil** | **(Verbindung Nr.) µg/g** |
|---|---|---|
| *Acorus calamus* | Rhizom | (2) 0.10 |
| *Angelica archangelica* | Wurzel | (9) 0.73; (1) 0.05; (2) 2.29 |
| *Amica montana, Arnica chamissonis* | Blüte | (3) 0.02; (2) 0.08 |
| *Cassia angustifolia, Cassia senna* | Frucht | (3) 1.22 |
| *Cola nitida* | Samen | (2) 0.11 |
| *Coriandrum sativum* | Frucht | (8) 1.43; (9) 3.96; (1) 0.03; (3) 1.75; (2) 1.24 |
| *Hedera helix* | Blatt | (4) 0.013; (2) 0.16; (5) 0.21; (6) 3.49; (7) 0.03 |
| *Hypericum perforatum* | Kraut | (1) 0.49 |
| *Lavandula spec.* | Blüte | (8) 0.72; (9) 3.67; (1) 0.36; (3) 0.54; (2) 4.36 |
| *Physostigma venenosum* | Frucht | (9) 0.82; (1) 0.08; (3) 0.19 |
| *Sambucus nigra* | Blüte | (8) 1.29; (9) 3.95; (2) 0.56; (1) 1.09; (3) 0.92 (2) 3.42 |
| *Silybum marianum* | Frucht | (5) 0.04 |
| *Theobroma cacao* | Samen | siehe Referenz 8 |
| *Thymus vulgaris, Thymus zygis* | Kraut | (2) 0.09 |

### Beispiele 2 - 4:

Um mögliche pharmakologische Eigenschaften der Verbindungen zu untersuchen, wurden die Verbindung Nr. 5 ((+)-N-[3',4'-dihydroxy-(E)-cinnamoyl]-L-Tryptophan; Kaffeesäure-L-Tryptophan als Beispiel für einen aliphatischen Aminosäurerest an R₆), und die erfindungsgemäße verbindung Nr. 8 ((+)- N-[3',4'-dihydroxy-(E)-cinnamoyl]-L-Asparaginsäure; Kaffeesäure-L-Aspartat als Beispiel für einen aromatischen Aminosäurerest an R₆) verwendet.

### Beispiel 2: Untersuchungen an einer menschlichen Leberzelllinie

Die HepG2-Zelllinie, Klon H20, wurde von Prof. Mersch-Sundermann, Universitat Giessen erhalten und wie in [2] beschrieben kultiviert. Die Zellen wurden in low glucose (1 g/l) Dulbecco's Modified Eagle's Medium (DMEM) mit L-Glutamine und 25 mM Hepes, das mit 15 % (v/v) hitzeinaktiviertem fötalen Kälberserum (FCS) und Gentamycin (30 µg/ml) versetzt war , in einer feuchten Atmosphäre bei 37+/-0.5°C, 5% CO₂ kultiviert. Die Zellen wurden trypsinisiert, mit PBS (pH 7.4) gewaschen, leicht zentrifugiert und anschließend im Zellmedium eine Zellsuspensionen hergestellt, indem das Sediment durch eine Kanüle gepresst wurde. Das Medium wurde alle drei bis fünf Tage ausgewechselt. Die zu testenden Verbindungen Nr. 5 und 8 wurden in einer Konzentration von 1 mg/ml in HepG2-Medium, dem statt FCS das serumfreie Supplement SerEx zugegeben wurde, gelöst und durch einen 0.2 µm Zelluloseazetatfilter filtriert.

Die Zellen wurden in 96-well Mikrotiterplatten (1 x 10⁴ Zellen/Well) ausgesät. Nach 24 h wurde das Medium entfernt und die Zellen wurden den zu testenden Verbindungen, in Konzentrationen von 100 und 10 µg/ml 48 h lang ausgesetzt. Die Zellstoffwechselaktivität wurde gemäß [3] mit 2.5 mg/ml MTT quantifiziert. Das extrazytosolische LDH [4] wurde mit einem Zytotoxizitätsassay quantifiziert.

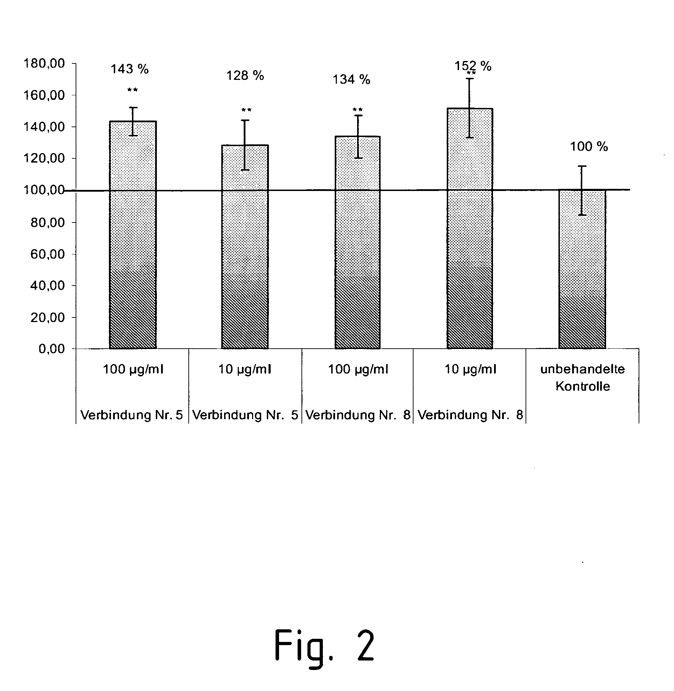

Sowohl die Verbindung Nr. 5 als auch die Verbindung Nr. 8 erhöhten die mitochondriale Aktivität nach 48-stündiger Inkubation (siehe Fig. 2). Eine verkürzte Inkubationszeit von 24 Stunden zeigte, dass Verbindung Nr. 8 den Energiestatus signifikant erhöhte, während Verbindung Nr. 5 keine Wirkung zeigte. Es konnte festgestellt werden, dass Verbindung Nr. 8 einen schnellen und intensiven stimulierenden Effekt aufweist. Die Bestimmung der LDH zeigte, dass keine nekrotische Zelltoxizität festzustellen war.

### Beispiel 3: Untersuchungen an einer menschlichen Keratinocyten-Zelllinie

Humane primäre Keratinocyten (NHK) wurden aus menschlicher Haut isoliert, die durch operative Resektion kaukasischer Patienten erhalten wurde. Es wurden in-vitro-Tests in Bezug auf die mitochondriale Aktivität [3], den BrdU-Einbau [5] und auf nekrotische Effekte mittels LDH-Assay [4] durchgeführt.

Für eine quantitative Real-Time-PCR wurden die NHK mit den zu testenden Verbindungen, die in serumfreien Keratinocyten-Medium gelöst waren, 6 Stunden lang inkubiert. Als Positivkontrolle wurde Keratinocyten-Medium mit verschiedenen Wachstumsfaktoren verwendet. Die Gesamt-RNA wurde mit dem Perfect RNA eukaryotic Mini Kit aufgereinigt. RNA-Aliquots wurden für Reverse Transkriptions-PCR (RT-PCR), die mit TaqMan Reverse Transcription Reagents® durchgeführt wurde, hergestellt.

Die quantitative RT-PCR wurde mit dem TaqMan Universal PCR Master Mix und spezifischen TaqMan Genexpressions-Assays für KGF, den KGF-Rezeptor, den EGF-Rezeptor, den InsulinRezeptor, STAT6 und 18srRNA als endogene Kontrolle in einem 7300 Real-Time PCR-System von Applied Biosystems durchgeführt.

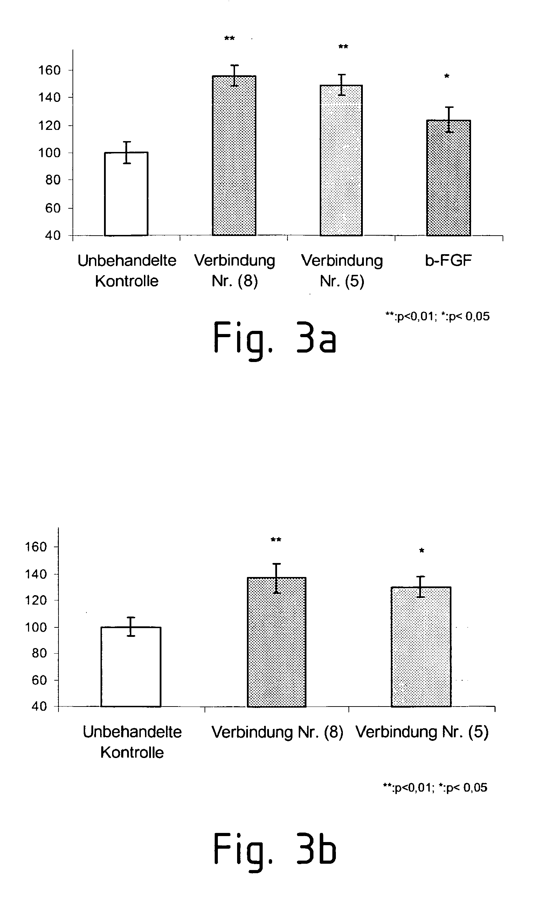

Die Untersuchungen wurden mit Zellen der 2. bis 6.Passage durchgeführt. Sowohl die Verbindung Nr. 5 als auch die Verbindung Nr. 8 erhöhten in einer Konzentration von 10 µg/mL sowohl die mitochondriale Aktivität als auch die Proliferation (Fig. 3). Eine Zelltoxizität wurde nicht festgestellt.

Da in vielen Fällen Auswirkungen auf die Physiologie von Keratinocyten durch eine erhöhte Expression von Wachstumsfaktoren oder Wachstumsfaktoren-Rezeptoren vermittelt wird, wurde der Einfluss der Verbindungen Nr. 5 und 8 auf die Genexpression des Keratinocyten-Wachstumsfaktors KGF, seines Rezeptors (KGFR), den Epidermalen Wachstumsfaktor-Rezeptor EGFR und den Insulinrezeptor InsR mittels quantitativer RT-PCR untersucht. Außerdem wurde die Expression des Transkriptionsfaktors STAT6 und des Gens für Involucrin (ein spezifisches Protein für die frühe ZellDifferenzierung) untersucht.

Es wurde keine erkennbare Wirkung auf die Expression von KGF, KGFR, EGFR, InsR und Involucrin beobachtet. Für STAT 6 wurde eine signifikant erhöhte Expression festgestellt (9 x höher im Vergleich zu den Kontrollen).

### Beispiel 4: Untersuchungen zur Adhäsion von Helicobacter pylori.

Adhäsionstests wurden gemäß [6, 7] durchgeführt. Dabei wurden FITC-markierte Bakterien mit den zu testenden Verbindungen (1 mg/ml) inkubiert. Deparaffinierte Magengewebsstücke wurden mit den Bakterien inkubiert. Mikroorganismen, die an dem Epithel anhaften, wurden unter dem Fluoreszenzmikroskop gezählt und mit einer nichtbehandelten Kontrolle verglichen

Die maximale Adhäsion, wie z. B. in den unbehandelten Kontrollgruppen (Negativkontrolle), festgestellt wurde, wurde mit einem Wert von +++++ belegt, während geringere Adhäsionen mit Werten von ++++, +++, ++, + oder - belegt wurden, wobei in der Positivkontrolle (Sialyllactose, [7]) ein Wert von - festgestellt wurde.

Insbesondere nach Inkubation der Bakterien mit Verbindung Nr. 8 (1 mg/mL) wurde ein starker und reproduzierbarer antiadhäsiver Effekt gegen *Helicobacter pylori* mit nahezu vollständiger Unterbindung der Adhäsion beobachtet, während Verbindung Nr. 5 keine Wirkung zeigte. Um eine direkte Cytotoxizität der zu testenden Verbindungen gegen *H. pylori* zu untersuchen, wurden die Verbindungen in Konzentrationen von 2,5 mg/mL in einem Scheibendiffusionsassay gegen den Mikroorganismus getestet (Positivkontrolle mit 0.5 µg Amoxicillin). Dabei wurden keine Anzeichen einer bakterioziden oder bakteriostatischen Wirkung der zu testenden Verbindungen festgestellt.

Die in den Beispielen 2 - 4 beschriebenen Versuche wurden auch mit der Verbindung Nr. 11 durchgeführt. Diese zeigte jedoch keinen der beschriebenen Effekte.

### Literatur

(1) Stark T, Hofmann T. Isolation, structure determination, synthesis, and sensory activity of N-phenylpropenoyl-L-amino acids from cocoa (Theobroma cocoa). J. Agric. Food Chem. 2005; 53: 5419-5428
(2) Dauer A, Hensel A, Lhoste F, Knasmueller S, Mersch-Sundermann V. Genotoxic and antigenotoxic effects of catechin and tannins from the bark of Hamamelis virginiana L. in metabolically competent, human hepatoma cells (HepG2) using single cell electrophoresis. Phytochem. 2003; 63: 199-207
(3) Mosmann M. Rapid colorimetric assay for cellular growth and survival: applications to proliferation and cytotoxicity assays. J. Immun. Meth. 1983; 65: 55-63
(4) Martin A, Clynes M. Comparison of 5 microplate colorimetric assays for in vitro cytotoxicity testing and cell proliferation assays. Cytotechnol. 1993; 11: 49-58
(5) Porstmann T, Ternyk T, Avrameas S. Quantification of 5-bromo-2'-deoxyuridine into DNA: an enzyme immunoassay for the assessment of the lymphoid cell proliferative response. J. Immun. Meth. 1985; 82:
(6) Lengsfeld C, Deters A, Faller G, Hensel A. High molecular weight polysaccharides from black currant seeds inhibit adhesion of Helicobacter pylori to human gastric mucosa. Planta Med. 2004; 70: 620-626
(7) Lengsfeld C, Titgemeyer F, Faller G, Hensel A. Glycosylated compounds from okra inhibit adhesion of Helicobacter pylori to human gastric mucosa. J. Agric. Food Chem. 2004; 52: 1495-1503
(8) Stark T, Justus H, Hofmann T. A stable isotope dilution analysis (SIDA) for the quantitative determination of N-phenylpropenoyl-L-amino acids in coffee beverage and cocoa. J. Agric. Food Chem. 2006; 54: 2859-2867

### Zeichnungen

Fig. 1: Strukturformeln einiger N-Phenylpropenoyl-Aminosäuren
Fig. 2: Einfluss der Verbindungen Nr. 5 und Nr. 8 (10 und 100 µg/ml) auf die mitochondriale Aktivität menschlicher Leberzellen (HEPG2) nach 48-stündiger Inkubation.
   Als Meßwert ist in Fig. 2 die relative mitochondriale Dehydrogenase-Aktivität (unbehandelte Kontrolle = 100%) aufgetragen. Die Balken stellen die Mittelwerte eines repräsentativen Versuchs mit n = 10 dar.
Fig. 3: Einfluss der Verbindungen Nr. 5 und Nr. 8 (10 µg/ml) auf die mitochondriale Aktivität (A) und die mitotische Proliferation (B) von HaCaT Keratinocyten nach 60-stündiger Inkubation.
   Als Meßwert ist in Fig. 3A die relative mitochondriale Dehydrogenase-Aktivität (unbehandelte Kontrolle = 100%) und in Fig. 3b die relative mitochondriale Proliferationsrate (unbehandelte Kontrolle = 100%) aufgetragen.
   Die mitochondriale Aktivität wurde mit dem MTT Test untersucht und die Proliferation durch BrdU-Incorporations-ELISA. Die Balken stellen die Standardfehler mit n = 10 dar. Negativkontrolle: unbehandelte Zellen, Positivkontrolle: Fibroblasten-Wachstumsfaktor FGF.

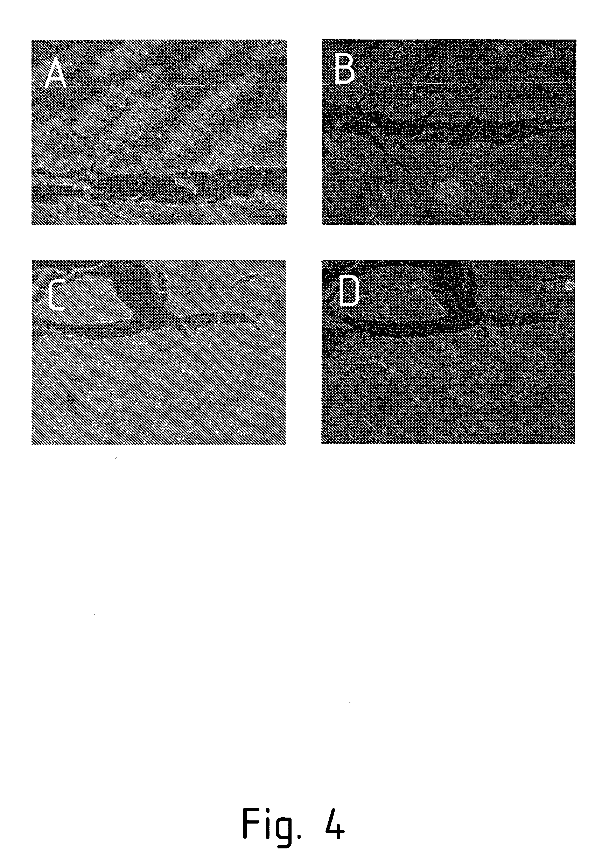

Fig. 4: Fluoresenzmikroskope (200 x) eines repräsentativen *in situ*-Experiments mit FITC-markierten *H. pylori* auf menschlicher Magenschleimhaut: (A) Vollständige Adhäsion (+++++) nichtbehandelter Bakterien, Fluoreszenzintensität auf 100 % standardisiert (Negativkontrolle), (B) Positivkontrolle (-), Fluoreszenzintensität 10% (C) Verbindung Nr. 5 (++++), Fluoreszenzintensität 78%, (D) Verbindung Nr. 8 (+) Fluoreszenzintensität 19%.

## Patentansprüche

1. Verbindungen der allgemeinen strukturformel 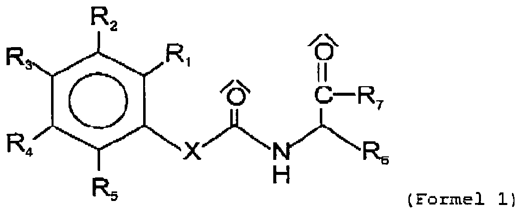 wobei es sich bei diesen Verbindungen, um E-konfigurierte N-Phenylpropenoyl-L-Aminosäurederivate handelt, und
wobei es sich bei X um einen HC=CH-Rest handelt,
wobei es sich bei R₅, R₄ und R₁ um Wasserstoffreste (H-)handelt,
wobei es sich bei R₃ um eine Hydroxigruppe (HO-) handelt,
wobei es sich bei R₃ um einen Wasserstoffrest (H-) oder eine Hydroxigruppe (HO-) handelt,
wobei es sich bei R₆ um einen -CH₂-COOH-Rest oder einen -CH₃-CH₂-COOH-Rest handelt, und
wobei es sich bei R₇ um eine Hydroxigruppe (-OH) handelt,
zur Verwendung in einem chirurgischen, therapeutischen oder diagnostischen Verfahren zur Behandlung des menschlichen oder tierischen Körpers.

2. Verwendung einer Verbindung gemäß einem der vorherigen Ansprüche zur Herstellung einem Arzneimittels zur Vorbeugung und/oder Behandlung von bakteriellen, viralen oder mykologischen Infektionen.

3. Verwendung einer Verbindung gemäß einem der vorherigen Ansprüche zur Herstellung eines Arzneimittels gegen orale Plaque.

4. Verwendung einer Verbindung gemäß einem der vorherigen Ansprüche als Nahrungsergänzungsmittel.

5. Verwendung einer Verbindung gemäß einem der vorherigen Ansprüche zur Verhinderung der Ausbildung von mikrobiellen Belägen auf Oberflächen.

6. Verwendung einer Verbindung gemäß einem der vorherigen Ansprüche als Haut- oder Mundpflegemittel.

7. Verwendung einer Verbindung gemäß einem der vorherigen Ansprüche, wobei diese Verbindung in Form eines wässrigen Extrakts, einer Lösung, einer Emulsion, einer Suspension, eines pulmonalen Inhalates, eines Implantates, einer Wasser/Öl-Emulsion, eine Öl/Wasser-Emulsion, eines Gels, einer Tablette, einer Kapsel, einer Creme, einer Salbe, eines Pflasters, einer Mikroemulsion, einer Nanoemulsion oder enkapsuliert in Liposomen, Micellen oder Microsphären vorliegt.

## Claims

1. Compounds having the general structural formula 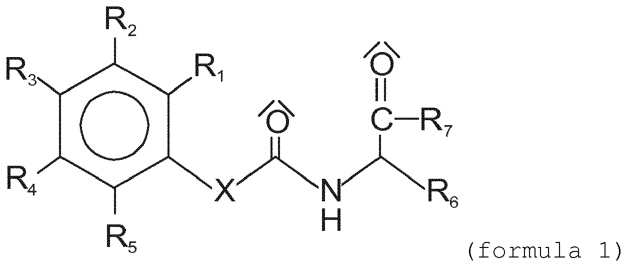 wherein said compounds are (E)-configured N-Phenylpropenoyl-L-amino acid derivatives and
wherein X is a HC=CH-moiety,
wherein R₅, R₄ and R₁ are hydrogen (H-) moieties,
wherein R₃ is a hydroxyl (OH-) group,
wherein R₂ is a hydrogen (H-) moiety or a hydroxyl (OH-) group,
wherein R₆ is a -CH₂-COOH-moiety or a -CH₂-CH₂-COOH-moiety, and
wherein R₇ is a hydroxyl (-OH) group,
for use in a surgical, therapeutically or diagnostic method for the treatment of the human or animal body.

2. Use of a compound according to any of the preceding claims for the manufacture of a pharmaceutical product for prophylaxis and/or the treatment of bacterial, viral or mycological infections.

3. Use of a compound according to any of the preceding claims for the manufacture of a pharmaceutical product against oral plaque.

4. Use of a compound according to any of the preceding claims as dietary supplement.

5. Use of a compound according to any of the preceding claims for prophylaxis against the development of microbial films on surfaces.

6. Use of a compound according to any of the preceding claims for skin or oral care products.

7. Use of a compound according to any of the preceding claims, wherein the compound is in the form of an aqueous extract, a solution, an emulsion, a suspension, a pulmonic inhalant, an implant, a water-in-oil emulsion, an oil-in-water emulsion, a gel, a tablet, a capsule, a crème, an ointment, a plaster, a micro-emulsion, a nano-emulsion or encapsulated in liposoms, micelles or microspheres.

## Revendications

1. Composés de la formule structurelle générale 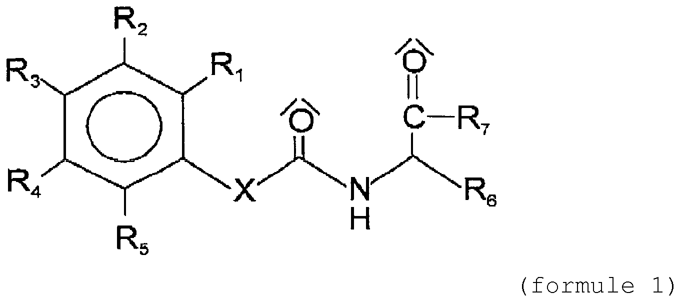 dans laquelle ces composés sont des dérivés d'acides N-phénylpropénoyl-aminés de forme L à configuration E et
X étant un radical HC=CH,
R₅, R₄ et R₁ sont des radicaux hydrogène (H-),
R₃ est un groupe hydroxy (HO-),
R₂ est un radical hydrogène (H-) ou un groupe hydroxy (HO-),
R₆ est un radical -CH₂-COOH ou un radical -CH₃-CH₂-COOH et
R₇ est un groupe hydroxy (-OH),
pour une utilisation dans un procédé chirurgical, thérapeutique ou diagnostique en vue du traitement du corps humain ou animal.

2. Utilisation d'un composé selon la revendication précédentes pour la fabrication d'un médicament de prévention et/ou de traitement d'infections bactériennes, virales ou mycologiques.

3. Utilisation d'un composé selon l'une des revendications précédente pour la fabrication d'un médicament contre la plaque dentaire.

4. Utilisation d'un composé selon l'une des revendications précédentes comme complément alimentaire.

5. Utilisation d'un composé selon l'une des revendications précédentes pour la fabrication d'un médicament destiné à empêcher la formation de couches microbiennes sur des surfaces.

6. Utilisation d'un composé selon l'une des revendications précédentes comme soin pour la peau ou comme produit pour l'hygiène buccale.

7. Utilisation d'un composé selon l'une des revendications précédentes, ce composé se présentant sous la forme d'un extrait aqueux, d'une solution, d'une émulsion, d'une suspension, d'un inhalant pulmonaire, d'un implant, d'une émulsion eau dans huile, d'une émulsion huile dans eau, d'un gel, d'une pastille, d'une gélule, d'une crème, d'une pommade, d'un emplâtre, d'une microémulsion, d'une nanoémulsion ou, encapsulé, dans des liposomes, des micelles ou des microsphères.
